# EUROPEAN PATENT APPLICATION

(11) **EP 3 988 079 A1**
(43) Date of publication of application: **27.04.2022**
(21) Application number: 20826882.1
(22) Date of filing: 22.06.2020
(51) Int. Cl.: A61K 8/37, A61K 8/34, A61Q 19/00, A61K 47/14, A61K 47/10, A61K 9/00, A61P 31/04

(54) **PRESERVATIVE FOR EXTERNALLY-APPLIED DERMAL PREPARATION, AND COSMETIC COMPOSITION AND PHARMACEUTICAL COMPOSITION EACH COMPRISING SAME**

(30) Priority: 21.06.2019 KR 20190074117
(71) Applicant: Activon Co., Ltd., Suwon-si, Gyeonggi-do 16681 (KR)
(72) Inventor: CHO, Youn Ki, Yongin-si, Gyeonggi-do 17005 (KR); PARK, Byung Gyu, Suwon-si, Gyeonggi-do 16707 (KR); CHO, Myung Chan, Yongin-si, Gyeonggi-do 16839 (KR); CHO, Se Hee, Seoul 07691 (KR); JUNG, Sung Won, Suwon-si, Gyeonggi-do 16705 (KR)
(74) Representative: Weickmann, Hans
(86) International application number: PCT/KR2020/008078
(87) International publication number: WO 2020/256519

(57) **Abstract**

Provided are a preservative for externally-applied dermal preparations including glyceryl undecylenate, glyceryl caprylate, and a glycol compound having 4 or 5 carbon atoms, and a cosmetic composition and a pharmaceutical composition, each including the same. A preservative for externally-applied dermal preparations according to one aspect may stably maintain a liquid state under room temperature and refrigerated conditions and may exhibit excellent antimicrobial effect even after formulation.

## Description

### TECHNICAL FIELD

The present disclosure relates to a preservative for externally-applied dermal preparations, and a cosmetic composition and a pharmaceutical composition, each including the same.

### BACKGROUND ART

Cosmetic compositions and pharmaceutical compositions for externally-applied dermal preparations cannot avoid microbial contamination during a preparation process and use thereof, and thus a preservative is used for the purpose of improving preservative activity of the products. Preservatives widely used are preservatives such as parabens (sodium parahydroxybenzoate), imidazolidinyl urea, phenoxyethanol, etc. These preservatives have high antimicrobial activity and excellent preservative activity, but are known to often cause toxicity, skin irritation, allergies, etc. Therefore, preservatives for externally-applied dermal preparations that do not cause skin irritation and have high preservative activity are being developed, and there is a continuous need to develop a safe preservative for externally-applied dermal preparations with superior preservative activity.

As part of these studies, preservatives for externally-applied dermal preparations using diols which are known to have antimicrobial activity have been studied. Korean Patent Publication No. 2013-0049729 discloses a synergistic ternary antimicrobial composition including a mixture of 2-phenoxyethanol; and at least two different alkanediols selected from the group consisting of 1,2-decanediol, 1,2-octanediol, 1,2-hexanediol, and 1,2-pentanediol. However, it is known that a large amount of alkanediol-based components in the ternary mixture has a disadvantage of causing toxicity, skin irritation, allergy, etc.

In view of this background, the present inventors have made intensive efforts to develop a preservative with excellent stability for externally-applied dermal preparations, and as a result, they found that a preservative with excellent antimicrobial or antiseptic activity for externally-applied dermal preparations may be prepared using a combination of glyceryl undecylenate, glyceryl caprylate, and glycols, thereby completing the present disclosure.

### DESCRIPTION OF EMBODIMENTS

### TECHNICAL PROBLEM

An aspect provides a preservative for externally-applied dermal preparations, the preservative including glyceryl undecylenate, glyceryl caprylate, and a glycol compound having 4 or 5 carbon atoms.

Another aspect provides a cosmetic composition including the preservative for externally-applied dermal preparations.

Still another aspect provides a pharmaceutical composition for externally-applied dermal preparations, the pharmaceutical composition including the preservative for externally-applied dermal preparations.

Still another aspect provides a method of preparing an externally-applied dermal preparation, the method including using the preservative for externally-applied dermal preparations as a preservative.

### SOLUTION TO PROBLEM

Hereinafter, the present disclosure will be described in more detail.

Unless defined otherwise, all technical terms used herein have the same meanings as those generally understood by one of ordinary skill in the art to which the present disclosure belongs. Further, although methods or samples are described herein, those similar or equivalent thereto are also incorporated in the scope of the present disclosure. The contents of all the publications disclosed as references herein are incorporated in the present disclosure.

An aspect provides a preservative for externally-applied dermal preparations, the preservative including glyceryl undecylenate, glyceryl caprylate, and a glycol compound having 4 or 5 carbon atoms.

As used herein, the term "externally-applied dermal preparation" is a concept that encompasses the overall composition applied to the skin, and it is a concept that includes, for example, various cosmetics such as basic cosmetics, makeup cosmetics, hair cosmetics, etc.; pharmaceutical compositions that are externally applied to the skin, including drugs and quasi-drugs, such as ointments, creams, lotions, etc.

As used herein, the term "antimicrobial" means resistance to all contaminating microorganisms such as bacteria, fungi, yeasts, etc., "antimicrobial activity" refers to a defensive power against these contaminating microorganisms, and "antiseptic activity" means a defensive power against decomposition by microbial contamination, and is a concept that includes antimicrobial activity.

As used herein, the term "preservative" refers to a substance that has antiseptic activity or antioxidant activity to prevent deterioration of a product for a long period of time and to maintain its original state, and "preservative activity" refers to a defensive power to prevent deterioration of a product for a long period of time and to maintain its original state.

In spite of effective antimicrobial activity, traditional preservatives have difficulties in mass production and processing due to instability induced during storage, for example, deterioration such as solidification, etc. Therefore, there is a need to develop a preservative with high stability at room temperature or refrigerated conditions along with effective antimicrobial activity.

The present inventors have tried to develop a natural product-derived preservative for externally-applied dermal preparations, the preservative capable of maintaining a liquid state, in particular, at room temperature and refrigerated conditions, in terms of convenience of processing and management, and securing superior antimicrobial activity and preservative activity, as compared to the traditional preservatives. According to one embodiment, a preservative for externally-applied dermal preparations including a combination of glyceryl undecylenate, glyceryl caprylate, and a glycol compound having 2 to 10 carbon atoms may exist in a liquid state at a low temperature condition of -18°C (see Experimental Example 1). In addition, a combination of glyceryl undecylenate, glyceryl caprylate, and pentylene glycol showed excellent antimicrobial activity against bacteria, yeasts, and fungi, and these effects were also effectively exhibited after formulation (see Experimental Examples 2 and 3).

In addition, according to one embodiment, in the preservative for externally-applied dermal preparations of a ternary composition including glyceryl undecylenate, glyceryl caprylate, and a glycol compound having carbon atoms, antimicrobial activity and storage stability may be controlled at the same time by changing the number of carbon atom of the glycol and content thereof. Based on this result, it was confirmed that it is possible to provide a preservative for externally-applied dermal preparations having excellent antimicrobial activity and excellent storage stability at the same time by controlling the content of glycols and a ratio of glyceryl undecylenate and glyceryl caprylate (see Experimental Example 4).

In one specific embodiment, the glycol compound may be butylene glycol or pentylene glycol, for example, pentylene glycol. Further, the glycol compound may be obtained by a method known by a person of ordinary skill in the art, for example, may be derived from a natural product. The glycol compound derived from a natural product may contribute to further minimizing irritation to the skin.

In the present disclosure, room temperature may refer to 20°C to 25 °C conditions, and refrigeration may refer to 4 °C to 10 °C conditions.

In one specific embodiment, the glyceryl undecylenate and glyceryl caprylate, and the glycol compound having 4 or 5 carbon atoms may be included in a weight ratio of 9:1 to 7:3. For example, the weight ratio may be 9:1 to 8.5:1.5, 9:1 to 8:2, 9:1 to 7.5:2.5, 8.5:1.5 to 8:2, 8.5:1.5 to 7.5:2.5, 8.5:1.5 to 7:3, 8:2 to 7.5: 2.5, or 8:2 to 7:3. When the weight ratio is deviated from the above range, and thus the content of the glycol compound having 4 or 5 carbon atoms is too low, it is apprehended that solidification and storage stability may be deteriorated. When the content of the glycol compound having 4 or 5 carbon atoms is too high, the antimicrobial activity is reduced and the preservative may not function as a preservative.

In one specific embodiment, the glyceryl undecylenate and the glyceryl caprylate may be included in a weight ratio of 1:4 to 1:8. For example, the weight ratio may be 1:4 to 1:7.5, 1:4 to 1:7.0, 1:4 to 1:6.5, 1:4 to 1:6.0, 1:4 to 1:5.5, 1:4 to 1:5, 1:4 to 1:4.5, 1:5 to 1:8, 1:5 to 1:7.5, 1:5 to 1:7.0, 1:5 to 1:6.5, 1:5 to 1:6.0, 1:5 to 1:5.5, 1:6 to 1:8, 1:6 to 1:7.5, 1:6 to 1:7.0, or 1:6 to 1:6.5. When the weight ratio is deviated from the above range, and thus the content of the glyceryl undecylenate is too high, it is apprehended that the antimicrobial activity may decrease. When the content of the glyceryl undecylenate is too low, it is apprehended that stability may be reduced at refrigerated condition.

In one specific embodiment, the preservative for externally-applied dermal preparations may include 10 parts by weight to 20 parts by weight of glyceryl undecylenate, and 70 parts by weight to 80 parts by weight of glyceryl caprylate, and 10 parts by weight to 20 parts by weight of glycol compound having 4 or 5 carbon atoms. For example, the glyceryl undecylenate may be included in an amount of 10 parts by weight to 18 parts by weight, 10 parts by weight to 16 parts by weight, 10 parts by weight to 14 parts by weight, 10 parts by weight to 12 parts by weight, 12 parts by weight to 20 parts by weight, 12 parts by weight to 18 parts by weight, 12 parts by weight to 16 parts by weight, 12 parts by weight to 14 parts by weight, 14 parts by weight to 20 parts by weight, 14 parts by weight to 18 parts by weight, or 14 parts by weight to 16 parts by weight; the glyceryl caprylate may be included in an amount of 70 parts by weight to 78 parts by weight, 70 parts by weight to 76 parts by weight, 70 parts by weight to 74 parts by weight, 70 parts by weight to 72 parts by weight, 72 parts by weight to 80 parts by weight, 72 parts by weight to 78 parts by weight, 72 parts by weight to 76 parts by weight, 72 parts by weight to 74 parts by weight, 74 parts by weight to 80 parts by weight, 74 parts by weight to 78 parts by weight, or 74 parts by weight to 76 parts by weight; and the glycol compound having 4 or 5 carbon atoms may be included in an amount of 10 parts by weight to 18 parts by weight, 10 parts by weight to 16 parts by weight, 10 parts by weight to 14 parts by weight, 10 parts by weight to 12 parts by weight, 12 parts by weight to 20 parts by weight, 12 parts by weight to 18 parts by weight, 12 parts by weight to 16 parts by weight, 12 parts by weight to 14 parts by weight, 14 parts by weight to 20 parts by weight, 14 parts by weight to 18 parts by weight, or 14 parts by weight to 16 parts by weight.

In one specific embodiment, the preservative for externally-applied dermal preparations may include 10 % by weight to 20 % by weight of glyceryl undecylenate, and 70% by weight to 80 % by weight of glyceryl caprylate, and 10 % by weight to 20 % by weight of glycol compound having 4 or 5 carbon atoms, based on the total weight of the composition. For example, the glyceryl undecylenate may be included in an amount of 10 % by weight to 18 % by weight, 10 % by weight to 16 % by weight, 10 % by weight to 14 % by weight, 10 % by weight to 12 % by weight, 12 % by weight to 20 % by weight, 12 % by weight to 18 % by weight, 12 % by weight to 16 % by weight, 12 % by weight to 14 % by weight, 14 % by weight to 20 % by weight, 14 % by weight to 18 % by weight, or 14 % by weight to 16 % by weight; the glyceryl caprylate may be included in an amount of 70 % by weight to 78 % by weight, 70 % by weight to 76 % by weight, 70 % by weight to 74 % by weight, 70 % by weight to 72 % by weight, 72 % by weight to 80 % by weight, 72 % by weight to 78 % by weight, 72 % by weight to 76 % by weight, 72 % by weight to 74 % by weight, 74 % by weight to 80 % by weight, 74 % by weight to 78 % by weight, or 74 % by weight to 76 % by weight; and the glycol compound having 4 or 5 carbon atoms may be included in an amount of 10 % by weight to 18 % by weight, 10 % by weight to 16 % by weight, 10 % by weight to 14 % by weight, 10 % by weight to 12 % by weight, 12 % by weight to 20 % by weight, 12 % by weight to 18 % by weight, 12 % by weight to 16 % by weight, 12 % by weight to 14 % by weight, 14 % by weight to 20 % by weight, 14 % by weight to 18 % by weight, or 14 % by weight to 16 % by weight.

The preservative for externally-applied dermal preparations according to the present disclosure may be prepared and distributed in the form of a mixed powder of the ternary combination, or in the form of a mixed powder mixed with a carrier such as a diluent, etc., as needed, or may be purchased for the preparation of cosmetic compositions or drugs for externally-applied dermal preparations, and used as a preservative. The preparation of the mixed powder may be appropriately performed by a person of ordinary skill in the art using known knowledge.

Another aspect provides a cosmetic composition including the preservative for externally-applied dermal preparations.

The cosmetic composition may be provided as a cosmetic composition with high preservative activity and safety by including the preservative for externally-applied dermal preparations. A content of the preservative for externally-applied dermal preparations used in the cosmetic composition is not particularly limited, as long as the desired preservative activity may be secured, and a person skilled in the art may appropriately determine the content according to the degree of the desired preservative activity. In one embodiment, the preservative for externally-applied dermal preparations may be included in an amount of 0.01 % by weight to 10 % by weight, 0.1 % by weight to 10 % by weight, 0.1 % by weight to 5 % by weight, or 0.1% by weight to 2 % by weight, based on the total weight of the cosmetic composition.

The cosmetic composition may have any formulation known as cosmetic compositions, for example, one formulation selected from the group consisting of a toner, an emulsion, a cream, a sun block, a foundation, an essence, a gel, a pack, a mask pack, a foam cleanser, a body cleanser, a softening lotion, an eyeliner, a shampoo, a conditioner, a soap, a hair fixative, a hair tonic, a hair cream, a hair styling gel, a lubricant, a toothpaste, and a wet tissue, but is not limited thereto.

Still another aspect provides a pharmaceutical composition for externally-applied dermal preparations including the preservative for externally-applied dermal preparations.

The pharmaceutical composition for externally-applied dermal preparations may be provided as a pharmaceutical composition with high preservative activity and safety, which is applicable to the skin as an external preparation, by including the preservative for externally-applied dermal preparations. A content of the preservative for externally-applied dermal preparations used in the pharmaceutical composition for externally-applied dermal preparations is not particularly limited as long as the desired preservative activity may be secured, and a person skilled in the art may appropriately determine the content according to the degree of the desired preservative activity. For example, the preservative for externally-applied dermal preparations may be included in an amount of 0.01 % by weight to 10 % by weight, 0.1% by weight to 10 % by weight, 0.1% by weight to 5 % by weight, or 0.1 % by weight to 2 % by weight, based on the total weight of the pharmaceutical composition for externally-applied dermal preparations.

The pharmaceutical composition for externally-applied dermal preparations may have any formulation known as externally-applied dermal preparations, for example, one formulation selected from the group consisting of an emplastra, a liniment, an ointment, a paste, a cataplasma, a suspension, an emulsion, a lotion, an aerosol, and a suppository, but is not limited thereto.

The pharmaceutical composition for externally-applied dermal preparations may be for systemic action of an active ingredient by skin application, or may be for topical action of an active ingredient by skin application. In one specific embodiment, the pharmaceutical composition may be for topical action of an active ingredient by skin application.

The cosmetic composition and the pharmaceutical composition for externally-applied dermal preparations may further include, in addition to the preservative for externally-applied dermal preparations, various known additives in the range that does not impair the effect of the preservative for externally-applied dermal preparations according to the formulation. According to one embodiment, the composition may further include an additive selected from the group consisting of a carrier, an emulsifier, a moisturizer, a skin conditioning agent, a surfactant, a chelating agent, an antioxidant, a germicide, a stabilizer, and any combination thereof.

The carrier may include animal fiber, vegetable fiber, wax, paraffin, starch, tragacanth, cellulose derivatives, polyethylene glycol, silicone, bentonite, silica, talc, zinc oxide, lactose, silica, aluminum hydroxide, calcium silicate, polyamide powder, water, ethanol, isopropanol, ethyl carbonate, ethyl acetate, benzyl alcohol, benzyl benzoate, propylene glycol, glycerol aliphatic ester, polyethylene glycol, a liquid diluent, a suspending agent such as ethoxylated isostearyl alcohol, polyoxyethylene sorbitol ester, and polyoxyethylene sorbitan ester, microcrystalline cellulose, aluminum metahydroxide, bentonite, agar or tragacanth, aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamido betaine, fatty alcohol, fatty acid glyceride, fatty acid diethanolamide, vegetable oil, linoleic derivatives, ethoxylated glycerol fatty acid ester, etc., but is not limited thereto.

The emulsifier includes liquid paraffin, cetyl octanoate, stearic acid, etc., but is not limited thereto.

The moisturizer includes glycerin, glyceryl stearate, etc., but is not limited thereto.

The skin conditioning agent includes cyclomethicone, dimethicone, etc., but is not limited thereto.

The surfactant may include cetostearyl alcohol, triethanolamine, carboxyvinyl polymer, etc., but is not limited thereto.

The chelating agent includes ethylenediaminetetrasodium acetic acid (EDTA), α-hydroxy fatty acid, lactoferrin, α-hydroxy acid, citric acid, lactic acid, malic acid, bilirubin, biliverdin, etc., but is not limited thereto.

The antioxidant may include butylhydroxyanisole, dibutylhydroxytoluene, propyl gallate, etc., but is not limited to.

In addition, components which may be blended with the cosmetic composition and the pharmaceutical composition may include oil and fat ingredients, emollients, organic and inorganic pigments, organic powders, ultraviolet absorbers, pH adjusters, alcohols, pigments, fragrances, blood circulation promoters, cooling agents, antiperspirant agents, etc.

Still another aspect provides a method of preparing an externally-applied dermal preparation, the method including using the preservative for externally-applied dermal preparations as a preservative.

The descriptions of the preservative for externally-applied dermal preparations, cosmetic composition, and pharmaceutical composition for externally-applied dermal preparations according to an aspect of the present disclosure are also applied to a detailed description of the method of preparing the externally-applied dermal preparation.

In one specific embodiment, the externally-applied dermal preparation may be a cosmetic composition or a pharmaceutical composition for externally-applied dermal preparations.

The preservative for externally-applied dermal preparations may be used in an amount of 0.01 % by weight to 10 % by weight with respect to the cosmetic composition or the pharmaceutical composition for externally-applied dermal preparations.

### ADVANTAGEOUS EFFECTS OF DISCLOSURE

A preservative for externally-applied dermal preparations according to one aspect may stably maintain a liquid state at room temperature and refrigerated conditions, and may exhibit excellent effective antimicrobial effects even after formulation, and therefore, its application to aqueous formulations is not limited.

In addition, the preservative for externally-applied dermal preparations according to one aspect may exhibit superior antiseptic activity, as compared to traditional parabens preservatives, while minimizing the content of alkanediol-based ingredients that may cause problems such as skin irritation, etc.

Accordingly, when the preservative for externally-applied dermal preparations is used, it is possible to prepare a cosmetic composition and a pharmaceutical composition having high preservative activity and safety, which are aqueous formulations.

### MODE OF DISCLOSURE

Hereinafter, the present disclosure will be described in more detail with reference to exemplary embodiments. However, these exemplary embodiments are only for illustrating the present disclosure, and the scope of the present disclosure is not limited to these exemplary embodiments.

Experimental Example 1. Evaluation of stability under low temperature condition

In this Experimental Example, stability, specifically, maintenance of a liquid state of a preservative for externally-applied dermal preparations according to one embodiment at a low temperature of -18 °C was evaluated. To this end, preservatives for externally-applied dermal preparations including glyceryl undecylenate, and glyceryl caprylate as essential components, and further including glycol, butylene glycol, or pentylene glycol were prepared. In detail, the preservatives for externally-applied dermal preparations were prepared according to components and contents shown in Table 1 below, and then low-temperature stability was evaluated after storage at a low temperature (-18 °C) for 72 hours.

As a result, as shown in Table 1, a combination of glyceryl undecylenate, glyceryl caprylate, and pentylene glycol, in particular, the preservative for externally-applied dermal preparations of Example 1 existed in a liquid state at a low temperature of -18 °C. These experimental results indicate that the storage stability problem pointed out as a traditional problem may be solved by changing the number of carbon atoms of glycol.

**[Table 1]**

| NAME OF RAW MATERIAL | EXAMPLE 1 | EXAMPLE 2 | EXAMPLE 3 | COMPAR ATIVE EXAMPLE 1 | COMPAR ATIVE EXAMPLE 2 | COMPAR ATIVE EXAMPLE 3 | COMPAR ATIVE EXAMPLE 4 | COMPAR ATIVE EXAMPLE 5 | COMPAR ATIVE EXAMPLE 6 | COMPAR ATIVE EXAMPLE 7 | COMPAR ATIVE EXAMPLE 8 | COMPAR ATIVE EXAMPLE 9 | COMPAR ATIVE EXAMPLE 10 | COMPAR ATIVE EXAMPLE 11 | COMPAR ATIVE EXAMPLE 12 |
|---|---|---|---|---|---|---|---|---|---|---|---|---|---|---|---|
| GLYCERYL CAPRYLATE | 42.5 | 42.5 | 42.5 | 51 | 51 | 51 | 59.5 | 59.5 | 59.5 | 68 | 68 | 68 | 76.5 | 76.5 | 76.5 |
| GLYCERYL UNDECYLENATE | 7.5 | 7.5 | 7.5 | 9 | 9 | 9 | 10.5 | 10.5 | 10.5 | 12 | 12 | 12 | 13.5 | 13.5 | 13.5 |
| PENTYLENE GLYCOL | 50 | | | 40 | | | 30 | | | 20 | | | 10 | | |
| PROPYLENE GLYCOL | | 50 | | | 40 | | | 30 | | | 20 | | | 10 | |
| BUTYLENE GLYCOL | | | 50 | | | 40 | | | 30 | | | 20 | | | 10 |
| EVALUATION | 0 | × | × | △ | × | × | × | × | × | × | × | × | × | × | × |

| | | | | | | | | | | | | | | | |
|---|---|---|---|---|---|---|---|---|---|---|---|---|---|---|---|
| 0. LIQUID STATE △: PARTIALLY SOLIDIFIED × : SOLIDIFIED (UNIT: WT%) | | | | | | | | | | | | | | | |

### Experimental Example 2. Evaluation of antimicrobial activity

In this Experimental Example, antimicrobial activities against bacteria, yeasts, and fungi were evaluated on *E.coli* (ATCC 8739), *Pseudomonas aeruginosa* (ATCC 9027), *Staphylococcus aureus* (ATCC 6538), *Candida albicans* (ATCC 10231), and *Aspergillus niger* (ATCC 16404).

### 2-1. Evaluation of antimicrobial activity of single ingredient

Antimicrobial activity of glyceryl undecylenate, glyceryl caprylate, pentylene glycol, propylene glycol, or butylene glycol was evaluated, respectively. Samples of various concentrations (0.01% to 10%) prepared by 12-step serial 1/10 dilutions of 10% each test material were prepared, and then, bacteria, yeasts, or fungi were seeded at a concentration of 10⁶/ml in 10 ml of a liquid culture solution prepared by adding each sample to a tryptic soy broth (TSB), followed by incubation at 32°C for 24 hours.

Thereafter, the state of bacteria, etc. was observed, and a concentration that significantly inhibited the number of the seeded initial strains was determined as a minimum inhibitory concentration (MIC), and the results are shown in Table 2. As a result, as shown in Table 2, glyceryl undecylenate, glyceryl caprylate, or pentylene glycol showed excellent antimicrobial activity.

**[Table 2]**

| TEST MATERIAL | *E. coli* | *P. aeruginosa* | *S*. *aureus* | *C. albicans* | *A. niger* |
|---|---|---|---|---|---|
| GLYCERYL CAPRYLATE | 1.5 | <3.0 | 1.5 | 1.0 | 2.0 |
| GLYCERYL UNDECYLENATE | 1.0 | <2.0 | 1.5 | 1.5 | 2.0 |
| PENTYLENE GLYCOL | 3.0 | 5.0 | 5.0 | 5.0 | 3.0 |
| PROPYLENE GLYCOL | <10.0 | <10.0 | <10.0 | <10.0 | <10.0 |
| BUTYLENE GLYCOL | <10.0 | <10.0 | <10.0 | <10.0 | <10.0 |
| | | | | (UNIT: %) | |

### 2-2. Evaluation of antimicrobial activity of ternary composition for externally-applied dermal preparation

Based on the results of the antimicrobial activity test in Table 2, antimicrobial activity of a combination of glyceryl undecylenate, glyceryl caprylate, and a glycol compound according to the components and contents shown in Table 3 below was evaluated. A detailed experiment was performed in the same manner as in Experimental Example 2-1.

**[Table 3]**

| NAME OF RAW MATERIAL (UNIT: WT%) | FORMULATION EXAMPLE | | | | | |
|---|---|---|---|---|---|---|
| | A | B | C | D | E | F |
| PURIFIED WATER | To 100 | To 100 | To 100 | To 100 | To 100 | To 100 |
| GLYCERYL CAPRYLATE | 1.275 | 1.275 | 1.275 | 0.75 | 0.75 | 0.75 |
| GLYCERYL UNDECYLENATE | 0.225 | 0.225 | 0.225 | 0.75 | 0.75 | 0.75 |
| PENTYLENE GLYCOL | 1.5 | | | 1.5 | | |
| PROPYLENE GLYCOL | | 1.5 | | | 1.5 | |
| BUTYLENE GLYCOL | | | 1.5 | | | 1.5 |

Table 4 shows the results of evaluating antimicrobial activity of the combination of glyceryl undecylenate, glyceryl caprylate, and the glycol compound. As shown in Table 4 below, Formulations A and D having the ternary composition of glyceryl undecylenate, glyceryl caprylate, and pentylene glycol exhibited significant antimicrobial activity at lower concentrations than other formulations. In particular, Formulation A, in which the content of glyceryl caprylate was more dominant, showed excellent antimicrobial activity, as compared with Formulation D, in which glyceryl caprylate and glyceryl undecylenate were mixed at a ratio of 1:1.

**[Table 4]**

| TEST MATERIAL | *E. coli* | *P. aeruginosa* | *S. aureus* | *C. albicans* | *A. niger* |
|---|---|---|---|---|---|
| FORMULATION A | 1.5 | 1.0 | 1.0 | 1.2 | 1.0 |
| FORMULATION B | 3.0 | 2.0 | 1.5 | 3.0 | 2.5 |
| FORMULATION C | 2.5 | 2.0 | 1.5 | 3.0 | 2.5 |
| FORMULATION D | 1.8 | 2.0 | 1.5 | 1.5 | 1.5 |
| FORMULATION E | 3.0 | 2.0 | 1.8 | 3.5 | 3.0 |
| FORMULATION F | 2.5 | 2.0 | 1.8 | 3.5 | 3.0 |
| | | | | | (UNIT: %) |

Based on the results of the antimicrobial activity test in Table 4, antimicrobial activity of a combination of glyceryl undecylenate, glyceryl caprylate, and pentylene glycol having the components and according to shown in Table 5 below was evaluated. A detailed experiment was performed in the same manner as in Experimental Example 2-1.

**[Table 5]**

| NAME OF RAW MATERIAL (UNIT: WT%) | FORMULATION EXAMPLE | | | |
|---|---|---|---|---|
| | A | B | C | D |
| PURIFIED WATER | To 100 | To 100 | To 100 | To 100 |
| GLYCERYL CAPRYLATE | 1.275 | 1.000 | 0.750 | 0.225 |
| GLYCERYL UNDECYLENATE | 0.225 | 0.500 | 0.750 | 1.275 |
| PENTYLENE GLYCOL | 1.500 | 1.500 | 1.50 | 1.500 |

Table 6 shows the results of evaluating antimicrobial activity of the combination of glyceryl undecylenate, glyceryl caprylate, and pentylene glycol. As shown in Table 6 below, Formulations A to D having the ternary composition of glyceryl undecylenate, glyceryl caprylate, and pentylene glycol exhibited excellent antimicrobial activity at lower concentrations than single treatment with glyceryl undecylenate, glyceryl caprylate, or pentylene glycol. Further, like the above results, Formulations A and B, in which the content of glyceryl caprylate was more dominant than the content of glyceryl undecylenate, showed more excellent antimicrobial activity.

**[Table 6]**

| TEST MATERIAL | *E. coli* | *P. aeruginosa* | *S. aureus* | *C*. *albicans* | *A. niger* |
|---|---|---|---|---|---|
| FORMULATION A | 1.5 | 1.0 | 1.0 | 1.2 | 1.0 |
| FORMULATION B | 1.5 | 1.0 | 1.0 | 1.5 | 1.5 |
| FORMULATION C | 1.8 | 2.0 | 1.5 | 1.5 | 1.5 |
| FORMULATION D | 1.8 | 2.5 | 1.4 | 1.5 | 1.5 |
| FORMULATION E | 1.8 | 2.5 | 1.5 | 1.5 | 1.5 |

| | | | | | |
|---|---|---|---|---|---|
| (UNIT: %) | | | | | |

Experimental Example 3. Preparation of cosmetic composition and Evaluation of antiseptic activity

In this Experimental Example, the antiseptic activity in cosmetic compositions having an emulsion or body wash formulation was evaluated using a combination of glyceryl undecylenate, glyceryl caprylate, and pentylene glycol.

### 3-1. Evaluation of antiseptic activity of emulsion formulation

The antiseptic activities of emulsion formulations including a combination of glyceryl undecylenate, glyceryl caprylate, and the glycol compound according to the components and contents shown in Table 7 below were evaluated. Emulsion or body wash formulations were prepared in the same manner, except that no chemical preservative was used during preparation in Comparative Example 1 and a combination of ethylparaben and phenoxyethanol was used as a chemical preservative in Comparative Example 2.

Thereafter, each microbial solution of *E*. *coli* (ATCC 8739), *Staphylococcus aureus* (ATCC 6538), *Pseudomonas aeruginosa* (ATCC 99027) was added to 25 g of the cosmetic composition of the emulsion formulation at an initial concentration of 10⁶ cfu (colony forming unit)/g or more for each sample, followed by mixing. Subsequently, the number of viable cells was measured by taking 1 g of each cosmetic composition at intervals of 0 day, 7 days, 14 days, 21 days, and 28 days while culturing in an incubator at 30 °C to 32 °C for 4 weeks. In addition, a microbial solution of the fungus *Aspergillus niger* (ATCC 16404) was added at an initial concentration of 10⁵ cfu/g or more for each sample, followed by mixing. Then, the presence or absence of odor and hyphae and spore formation on the surface of the sample were observed at intervals of 7 days while culturing in an incubator at 25 °C. The evaluation was performed according to the following standards.
-: No odor and no hyphae and spores for 8 weeks, good
+: Fungus observed on the wall or lid within 4 weeks
++: Odor and fungus observed on a part of the surface within 4 weeks
+++: Odor and fungus observed on the entire surface within 4 weeks

**[Table 7]**

| NAME OF RAW MATERIAL (UNIT: WT%) | FORMULATION EXAMPLE | | COMPARATIVE EXAMPLE | |
|---|---|---|---|---|
| | 1 | 2 | 1 | 2 |
| PURIFIED WATER | To 100 | To 100 | To 100 | To 100 |
| CARBOMER 940 | 10 | 10 | 10 | 10 |
| POLYACRYLATE-13/POLYISOBUTENE/POLYSORBATE 20 | 0.8 | 0.8 | 0.8 | 0.8 |
| BUTYLENE GLYCOL | 15 | 15 | 15 | 15 |
| PEG 40 HYDROGENATED CASTOR OIL | 0.8 | 0.8 | 0.8 | 0.8 |
| PHENYL TRIMETHICONE | 1 | 1 | 1 | 1 |
| L-ARGININE | 0.16 | 0.16 | 0.16 | 0.16 |
| GLYCERYL UNDECYLENATE, GLYCERYL CAPRYLATE AND PENTYLENE GLYCOL (0.075:0.425:0.500) | 2.5 | - | - | - |
| GLYCERYL UNDECYLENATE, GLYCERYL CAPRYLATE AND PENTYLENE GLYCOL (0.01:0.40:0.500) | - | 2.5 | - | - |
| ETHYLPARABEN | - | - | - | 0.05 |
| PHENOXY ETHANOL | - | - | - | 0.9 |

Table 8 shows the results of evaluating the antiseptic activities of emulsion formulations including the combination of glyceryl undecylenate, glyceryl caprylate, and pentylene glycol. As shown in Table 8 below, both Formulation Examples 1 and 2 showed excellent antiseptic activity against bacteria and fungi. In addition, according to the results of the antiseptic activity test, it was confirmed that the cosmetic composition having the emulsion formulations including the combination of undecylenate, glyceryl caprylate, and pentylene glycol showed remarkably excellent antiseptic activity, as compared with those using ethylparaben and phenoxyethanol which are traditionally used cosmetic preservatives.

**[Table 8]**

| COSMETIC MATERIAL | | NUMBER OF BACTERIA (cfu/g) | | | | | FUNGUS |
|---|---|---|---|---|---|---|---|
| | | DAY 0 | DAY 7 | DAY 14 | DAY 21 | DAY 28 | |
| EMULSION | FORMULATION EXAMPLE 1 | 1 × 10⁶ | 0 | 0 | 0 | 0 | - |
| | FORMULATION EXAMPLE 2 | 1 × 10⁶ | <100 | <10 | 0 | 0 | - |
| | COMPARATIVE EXAMPLE 1 | 1 × 10⁶ | 3.5 × 10⁵ | 6.0 × 10⁵ | 1.1 × 10⁶ | 1.7 × 10⁶ | + + + |
| | COMPARATIVE EXAMPLE 2 | 1 × 10⁶ | <100 | <10 | <100 | <100 | - |

### 3-2. Evaluation of antiseptic activity of body wash formulation

The antiseptic activities of body wash formulations including a combination of glyceryl undecylenate, glyceryl caprylate, and glycol compound according to the components and contents shown in Table 9 below were evaluated. Body wash formulations were prepared in the same manner, except that no chemical preservative was used during preparation in Comparative Example 3 and a combination of ethylparaben and phenoxyethanol was used as a chemical preservative in Comparative Example 4.

**[Table 9]**

| NAME OF RAW MATERIAL (UNIT: WT%) | FORMULATION EXAMPLE | | COMPARATIVE FORMULATION EXAMPLE | |
|---|---|---|---|---|
| | 3 | 4 | 3 | 4 |
| PURIFIED WATER | To 100 | To 100 | To 100 | To 100 |
| DISODIUM EDTA | 0.1 | 0.1 | 0.1 | 0.1 |
| POLYACRYLATE-13/POLYISOBUTENE/POLYSORBATE 20 | 8 | 8 | 8 | 8 |
| SODIUM LAURETH SULFATE | 9 | 9 | 9 | 9 |
| PEG 80 SORBITAN LAURATE | 15 | 15 | 15 | 15 |
| PEG 40 HYDROGENATED CASTOR OIL | 0.8 | 0.8 | 0.8 | 0.8 |
| COCAMIDOPROPYL BETAINE | 19 | 19 | 19 | 19 |
| SODIUM CHLORIDE | 0.8 | 0.8 | 0.8 | 0.8 |
| GLYCERYL UNDECYLENATE, GLYCERYL CAPRYLATE AND PENTYLENE GLYCOL (0.075:0.425:0.500) | 2.5 | - | - | - |
| GLYCERYL UNDECYLENATE, GLYCERYL CAPRYLATE AND PENTYLENE GLYCOL (0.01:0.40:0.500) | - | 2.5 | - | - |
| ETHYLPARABEN | - | | - | 0.05 |
| PHENOXYETHANOL | - | | - | 0.9 |

Table 10 shows the results of evaluating the antiseptic activities of body wash formulations including the combination of glyceryl undecylenate, glyceryl caprylate, and pentylene glycol. As shown in Table 9 below, both Formulation Examples 3 and 4 showed excellent antiseptic activity against bacteria and fungi. In addition, according to the results of the antiseptic activity test, it was confirmed that the cosmetic composition having the body wash formulations including the combination of undecylenate, glyceryl caprylate, and pentylene glycol showed remarkably excellent antiseptic activity, as compared with those using ethylparaben and phenoxyethanol which are traditionally used cosmetic preservatives.

**[Table 10]**

| COSMETIC MATERIAL | | NUMBER OF BACTERIA (cfu/g) | | | | | FUNGUS |
|---|---|---|---|---|---|---|---|
| | | DAY 0 | DAY 7 | DAY 14 | DAY 21 | DAY 28 | |
| BODY WASH | FORMULATION EXAMPLE 3 | 1 × 10⁶ | <10 | 0 | 0 | 0 | - |
| | FORMULATION EXAMPLE 4 | 1 × 10⁶ | <10 | <10 | 0 | 0 | - |
| | | | | | | | |
| | COMPARATIVE EXAMPLE 3 | 1 × 10⁶ | 3.0 × 10⁵ | 5.0 × 10⁵ | 1.0 × 10⁶ | 1.5 × 10⁶ | + + + |
| | COMPARATIVE EXAMPLE 4 | 1 × 10⁶ | <100 | <10 | <10 | <10 | - |

The above experimental results taken together, the compositions for externally-applied dermal preparations including the combination of glyceryl undecylenate, glyceryl caprylate, and pentylene glycol may stably maintain a liquid state under low temperature conditions, and may exhibit excellent antimicrobial effect even after formulation, and thus they are useful as externally-applied dermal preparations such as cosmetic compositions, etc.

Experimental Example 4. Evaluation of efficacy according to changes in combination of ternary composition and content ratio

In this Experimental Example, based on the cosmetic composition of the emulsion formulation of Experimental Example 3-1, effects of changes in the number of carbon atoms of the glycol compound, the content ratio of the glycol compound, and the content ratio of the ternary composition on antimicrobial activity and storage stability were evaluated. In this Experimental Example, the cosmetic composition of the emulsion formulation was prepared to include 1.5% of the preservative for externally-applied dermal preparations of the ternary composition according to one embodiment. Regarding the antimicrobial activity, a detailed experimental procedure was performed in the same manner as in Experimental Example 2-1, except that *Aspergillus brasiliensis* (ATCC 16404) was used instead of *Aspergillus niger.* In addition, storage stability was evaluated after preparing cosmetic compositions including the preservative for externally-applied dermal preparations of the ternary composition according to one embodiment, and storing the cosmetic compositions at room temperature and/or refrigerated (5 °C) conditions for 72 hours. Meanwhile, glyceryl caprylate was expressed as GMCY and glyceryl undecylenate was expressed as GMUD below.

### 4-1. Carbon atoms of glycols

Table 11 shows the results of evaluating the antimicrobial activity and storage stability according to changes in the number of carbon atoms of glycols. As shown in Table 11 below, it was found that the preservative for externally-applied dermal preparations including butylene glycol and pentylene glycol had superior antimicrobial activity and storage stability compared to those including other glycols. Specifically, it was found that butylene glycol was the most excellent in terms of storage stability and pentylene glycol was the most excellent in terms of antimicrobial activity.

**[Table 11]**

| | **ANTIMICROBIAL ACTIVITY (MIC)** | | | | | **STABILITY** | |
|---|---|---|---|---|---|---|---|
| **GMCY:GMUD: GLYCOL SOLVENT (76.5 : 13.5 : 10)** | **Bacteria (Gram-)** | | **Bacteria (Gram+)** | **Yeast** | **Mold** | **ROOM TEMPERATURE** | **REFRIGERATED** |
| | ***E. coli*** | ***P. aeruginos*** | ***S. aureus*** | ***C*. *albicans*** | ***A. brasiliensis*** | | |
| **Propanediol (C3)** | 0.5% | >10% | <0.06% | 0.125% | >10% | STABLE | SOLIDIFIED |
| **Butylene glycol (C4)** | 0.5% | >10% | <0.06% | <0.06% | 10% | STABLE | STABLE |
| **Pentylene glycol (C5)** | 0.5% | >10% | <0.06% | <0.06% | >10% | STABLE | SOLIDIFIED |
| **1,2-hexanediol (C6)** | 0.5% | >10% | <0.06% | 0.125% | >10% | STABLE | SOLIDIFIED |

### 4-2. Content ratio of glycols

Table 12 shows the results of evaluating the antimicrobial activity and storage stability according to changes in the content ratio of glycols. As shown in Table 12 below, it was found that as the content of glycols increased, storage stability tended to increase and antimicrobial activity tended to decrease. These experimental results indicate that storage stability may be improved by increasing the content of glycols under conditions in which a certain degree of antimicrobial activity was exhibited.

**[Table 12]**

| | **ANTIMICROBIAL ACTIVITY (MIC)** | | | | | **STABILITY** | |
|---|---|---|---|---|---|---|---|
| **[GMCY:GMUD] : PENTYLENE GLYCOL SOLVENT** | **Bacteria (Gram-)** | | **Bacteria (Gram+)** | **Yeast** | **Mold** | **ROOM TEMPERATURE** | **REFRIGERATED** |
| | ***E. coli*** | ***P. aeruginos*** | ***S*. *aureus*** | ***C*. *albicans*** | ***A. brasiliensis*** | | |
| **50 : 50** | 1% | >10% | 0.25% | 0.25% | >10% | STABLE | STABLE |
| **70 : 30** | 0.5% | 8% | 0.125% | 0.25% | >10% | STABLE | STABLE |
| **90 : 10** | 0.5% | >10% | <0.06% | <0.06% | >10% | STABLE | SOLIDIFIED |

### 4-3. Content ratio of ternary composition

Table 13 shows the results of evaluating the antimicrobial activity and storage stability according to changes in the content ratio of ternary composition. As shown in Table 13 below, it was found that storage stability may be improved by increasing the content of glycols (e.g., a ratio of 75:5:20), like the above results. It was also found that when the content ratio of GMCY: GMUD is about 5:1, storage stability may be improved under conditions in which the content of glycols is not increased, i.e., under conditions in which antimicrobial activity is maintained at a high state.

**[Table 13]**

| | | **ANTIMICROBIAL ACTIVITY (MIC)** | | | | | **STABILITY** | |
|---|---|---|---|---|---|---|---|---|
| | **GMCY:GMUD PENTYLENE GLYCOL SOLVENT** | **Bacteria (Gram-)** | | **Bacteria (Gram+)** | **Yeast** | **Mold** | **ROOM TEMPERA TURE** | **REFRIGERATED** |
| | | ***E. coli*** | ***P. aeruginos*** | ***S. aureus*** | ***C. albicans*** | ***A. brasiliensis*** | | |
| **GMCY: PENTYLENE GLYCOL COMPARISON** | **75 : 5 :20** | 0.5% | 10% | 0.125% | 0.25% | >10% | STABLE | STABLE |
| | **80 : 5: 15** | 0.5% | >10% | 0.125% | 0.25% | >10% | STABLE | SOLIDIFIED |
| | **85 : 5 : 10** | 0.5% | >10% | <0.06% | 0.125% | >10% | STABLE | SOLIDIFIED |
| **GMCY:GMUD COMPARISON** | **80 :10 : 10** | 0.5% | >10% | <0.06% | <0.06% | >10% | STABLE | SOLIDIFIED |
| | **76.5 : 13.5 : 10** | 0.5% | >10% | <0.06% | <0.06% | > 10% | STABLE | STABLE |
| | **75 : 15 : 10** | 0.5% | >10% | <0.06% | <0.06% | 10% | STABLE | STABLE |

The above-described description of the present disclosure is for illustrating, and it will be understood by those skilled in the art that the present disclosure may be implemented in a different specific form without changing the technical spirit or essential characteristics thereof. Therefore, it should be understood that the above embodiment is not limitative, but illustrative in all aspects.

## Claims

1. A preservative for externally-applied dermal preparations, the preservative comprising glyceryl undecylenate, glyceryl caprylate, and a glycol compound having 4 or 5 carbon atoms.

2. The preservative for externally-applied dermal preparations of claim 1, wherein the glycol compound is butylene glycol or pentylene glycol.

3. The preservative for externally-applied dermal preparations of claim 1, wherein the glycol compound is pentylene glycol.

4. The preservative for externally-applied dermal preparations of claim 1, wherein the glyceryl undecylenate and the glyceryl caprylate, and the glycol compound having 4 or 5 carbon atoms are comprised in a weight ratio of 9:1 to 7:3.

5. The preservative for externally-applied dermal preparations of claim 1, wherein the glyceryl undecylenate and the glyceryl caprylate are comprised in a weight ratio of 1:4 to 1:8.

6. The preservative for externally-applied dermal preparations of claim 1, comprising 10 parts by weight to 20 parts by weight of the glyceryl undecylenate, 70 parts by weight to 80 parts by weight of the glyceryl caprylate, and 10 parts by weight to 20 parts by weight of the glycol compound having 4 or 5 carbon atoms.

7. A cosmetic composition comprising the preservative for externally-applied dermal preparations of any one of claims 1 to 6.

8. The cosmetic composition of claim 7, wherein the preservative for externally-applied dermal preparations is comprised in an amount of 0.01 % by weight to 10 % by weight with respect to the total weight of the cosmetic composition.

9. The cosmetic composition of claim 7, wherein the cosmetic composition has one formulation selected from the group consisting of a toner, an emulsion, a cream, a sun block, a foundation, an essence, a gel, a pack, a mask pack, a foam cleanser, a body cleanser, a softening lotion, an eyeliner, a shampoo, a conditioner, a soap, a hair fixative, a hair tonic, a hair cream, a hair styling gel, a lubricant, a toothpaste, and a wet tissue.

10. A pharmaceutical composition for externally-applied dermal preparations, the pharmaceutical composition comprising the preservative for externally-applied dermal preparations of any one of claims 1 to 6.

11. The pharmaceutical composition for externally-applied dermal preparations of claim 10, wherein the preservative for externally-applied dermal preparations is comprised in an amount of 0.01 % by weight to 10 % by weight with respect to the total weight of the pharmaceutical composition for externally-applied dermal preparations.

12. The pharmaceutical composition for externally-applied dermal preparations of claim 10, wherein the pharmaceutical composition has one formulation selected from the group consisting of an emplastra, a liniment, an ointment, a paste, a cataplasma, a suspension, an emulsion, a lotion, an aerosol, and a suppository.
